# NEW EUROPEAN PATENT SPECIFICATION

(11) **EP 2 206 529 B2**
(45) Date of publication and mention of the opposition decision: **27.05.2020**
(45) Mention of the grant of the patent: 28.11.2012
(21) Application number: 08021954.6
(22) Date of filing: 18.12.2008
(51) Int. Cl.: A61M 5/315, A61M 5/20

(54) **Medication delivery device and method of manufacturing a medication delivery device**
Arzneimittelabgabevorrichtung und Verfahren zur Herstellung einer Arzneimittelabgabevorrichtung
Dispositif d'administration médicale et procédé de fabrication du dispositif d'administration médicale

(43) Date of publication of application: 14.07.2010
(73) Proprietor: Sanofi-Aventis Deutschland GmbH, 65929 Frankfurt am Main (DE)
(72) Inventor: Brüggemann, Ulrich, 65926 Frankfurt am Main (DE); Tyce, Daniel, Rugby CV22 5RY (GB); Adams, Lee, Warwick Warwickshire CV34 6HG (GB); Holdgate, James, Crewe, Cheshire CW4 8BE (GB); Müller-Pathle, Stephan, DI., 65926 Frankfurt am Main (DE); Tubb, Andrew Ph. D., Crewe, Cheshire CW4 8BE (GB); Glenton, David, Warwick Warwickshire CV34 5DY (GB)
(74) Representative: Schmidt, Christian

(56) References cited:
- WO-A-2006/069455
- WO-A1-95/24233
- GB-A- 2 285 518
- US-A1- 2004 054 328
- US-A1- 2006 079 765
- US-A1- 2006 264 831
- US-A1- 2006 274 548
- US-A1- 2007 100 222
- US-A1- 2008 033 369
- WILSON, TRACY: "Introduction to How hte iPhone Works", 23 February 2008 (2008-02-23)

## Description

The present invention relates to a medication delivery device for the administration of a drug, for example insulin, where different dosage sizes can be set. Here, it is important that the size of the selected dose is clearly visible for the user.

In WO 97/30742 A1 a syringe is shown having a dose setting mechanism, a button which can be operated to inject a set dose, a switch operated at a time between the start and the completion of the injection and an electronic representation of parameters such as the magnitudes of the set dose and the latest injected dose. The syringe has a stop watch which is reset and started when the switch is operated. The status of the watch function is electronically represented and is reproduced in a display.

In US 6,482,185 B1 an injection device is shown, which comprises a pen with a syringe to be emptied. The dose expelled from the syringe is set by means of a dose setting device. For people with an impaired vision, the setting of the dose is rather difficult. Therefore, an external display apparatus is provided that may be attached to the pen. Via contacts and counter contacts, information about the dose is transmitted to the display apparatus and shown on a large display.

WO 2006/069455 A1 discloses a medication delivery device according to the preamble of claim 1.

It is an aim of the present invention defined in claim 1 to provide a medication delivery device, which provides improved operability for the user.

For this aim, a medication delivery device according to claim 1 is provided.

The light source provides illumination of the window on the front surface of the medication delivery device, which can be used to display information to a user. In order to achieve an improved readability for the user, the light source illuminates a display element and input means. Accordingly, operating the medication delivery device is more convenient for the user. Furthermore, an improved brightness or contrast of the displayed information reduces the risk of misreading. By illuminating the transparent window, operability of the medication delivery device is enhanced, especially when the medication delivery device is used under dimly lit conditions or by a visually impaired user.

The medication delivery device comprises a display element within the at least partially transparent window, wherein the light source is capable of backlighting the display element.

The display element on the front surface of the medication delivery device indicates information for the user of the device, a selected dosage, for example. The selected dosage can e.g. be set by input means via at least one operating button. In order to achieve an improved readability for the user, a light source illuminates the display element. The light source illuminates the display element on the backside of the display element, which can be constructed using transparent electrodes. As the light source can be activated only during operation of the medication delivery device, battery life time can be increased. The display element can be constructed as an LCD-panel. An LCD-panel is a power efficient and low-cost display type, which can be easily implemented in the medication delivery device. LCD-panels are fabricated as transparent substrates with polarization changing liquid crystals in between and illumination of the LCD-panel can be performed from the backside by the light source.

The medication delivery device comprises input means for operating the medication delivery device, wherein the at least partially transparent window is at least partially embedded into the input means.

The light source provides illumination of the window on the front surface of the medication delivery device, which is located on or embedded in input means on the front surface of the medication delivery device, so as to display information to a user about the specific function of respective input means. In order to achieve an improved operability for the user, the light source also illuminates the input means. Accordingly, operating the medication delivery device is more convenient for the user.

According to the invention, the medication delivery device comprises a light guide which is arranged in the optical path between the light source and the at least partially transparent window.

In this example, the light source can be located within the housing of the medication delivery device regardless of the positions of the display element and/or the input means. Accordingly, the construction of the medication delivery device is simplified, as the light source can be placed within the housing without constraints with respect to the transparent window. The light from the light source is distributed or guided by the light guide to the transparent window.

The light guide comprises a foil.

Illumination for larger sized windows, e.g. for display elements, can be provided by a transparent foil, which acts as a distributor and diffuser for the light being emitted by the light source. The foil is arranged underneath the display element and the input means. It is however also conceivable to arrange the foil such that the element and/or the input means are surrounded by the foil.

In addition, the foil can be coated with a reflective layer, for example a metal layer. The reflective layer embeds the transparent foil such that light can only escape at predefined positions e.g. those positions where the display element and/or the input means are located.

According to a further embodiment, the light source comprises at least two light emitting elements , preferably light-emitting diodes, capable of emitting light with different colors.

Different colors can attract the attention of a user of the medication delivery device. Accordingly, it is conceivable to use light with different colors for illuminating the display element and the input means, e.g. the operating button. It is also possible to selectively apply different light with different colors. For example, in case the medication delivery device is constructed with a plurality of operating buttons, buttons being associated with important functions can be illuminated with another color than those being used for other functions. It is also conceivable that colors may change during operation, so as to guide the user to operate a particular one of the operating buttons.

The light source is adapted to operate in a first state and in a second state.

For operating the medication delivery device, input from the user might be necessary which includes, for example, selecting the dosage of medication. Furthermore, a self-test of the medication delivery device can be performed, which includes, for example, detecting the charging condition of a battery. Accordingly, these different operating modes of the medication delivery device can correspond to a first state and a second state.

In one embodiment, the light source is adapted to provide light having a first color, preferably white or green, in the first state and the light source is adapted to provide light having a second color, preferably red or orange, in the second state.

According to this embodiment, the attention of a user is attracted to the actual mode of the medication delivery device which corresponds to the actual state of the light source. As the illumination of the medication delivery device changes, a user can quickly recognize the actual operating condition of the medication delivery device. As such, even visually impaired users can gather important information without being forced to read characters or the like on a display.

In one embodiment, the second state indicates a mode of configuration of the device during which a user input is required.

Operating of the medication delivery device can require an input from the user, for example, setting a prescribed dosage or the like. In order to facilitate these setup steps, the illuminating color of the medication delivery device can change, in order to indicate that a setup procedure is performed. As the appearance of the medication delivery device changes, a user can immediately recognize that the actual setup procedure of the medication delivery device is not yet completed, so as to assist a user during this step.

The second state of the light source indicates a configuration of the device requiring an input from a user or an alarm state, for example a low battery state of the device.

Occasionally, the user is required to exchange a battery or to recharge a battery. By the change in the illumination or color of the medication delivery device, the user can immediately recognize that the battery is in a low charging state. Hence, the user is assisted by directing his or her attention to the necessary procedures.

In one embodiment, the light source is adapted to provide a blinking illumination or a brighter illumination in the second state.

A blinking illumination or a brighter illumination can further enhance attracting the attention of a user of the medication delivery device. Accordingly, even visually impaired users are provided with important information about their medication delivery device.

According to a further embodiment, the light source is adapted to operate in a third state, in which the light source provides light with a different color with respect to the first and second color.

It is conceivable that different operating modes of the medication delivery device can be assigned to different colors, so as to guide the user when using the device. For example, setup procedures can utilize a display being illuminated by a yellow colored light, alarm states can be assigned to a red and normal operation to a green or white light. This improves operability of the medication delivery device and allows for guiding the user through the various operating modes.

According to a further embodiment, the light source comprises light-emitting-diodes or a multi-color light-emitting-diode arrangement. However, other types of light sources known to the person skilled in the art can be used as well.

A light-emitting-diode (LED) is a cost efficient and low-power light source, which can be easily implemented in the medication delivery device. As LEDs are delivered in various colors, including bi- and tri-colored types, a wide spectrum of possible illumination colors of the display element and/or the operating button can be achieved. Due to the low-power consumption, no unwanted heating of the medication delivery device occurs and battery life time improves.

According to a further embodiment, the medication delivery device comprises a further light source which is arranged in proximity to the at least partially transparent window.

A further light source which is arranged in proximity to the display element can further enhance attracting the attention of a user of the medication delivery device. Accordingly, even visually impaired users are provided with important information about their medication delivery device.

According to a preferred embodiment of the present invention, the medication delivery device is reusable. In this case a used or empty cartridge can be removed from the housing which accommodates the cartridge and be replaced by a new cartridge. Alternatively, the medication delivery device can be disposable.

Other features will become apparent from the following detailed description when considered in conjunction with the accompanying drawings.

Figure 1 is a schematical top view of a medication delivery device according to an embodiment.

Figure 2 is a schematical sectional view of a medication delivery device according to an embodiment.

Figure 3 is a schematical sectional view of a medication delivery device according to an embodiment not currently claimed.

Figures 4A and 4B each schematically show a top view around input means of a medication delivery device according to an embodiment.

Figures 5A to 5D each schematically show a top view of a display element in different operating modes according to an embodiment.

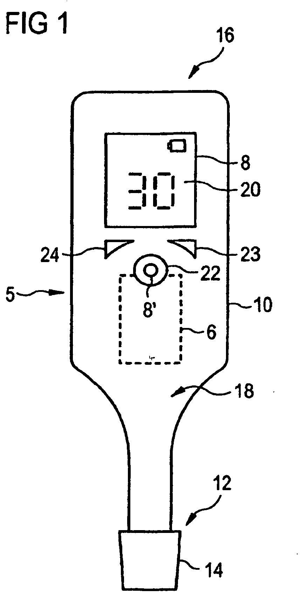

In figure 1 an embodiment of a medication delivery device 5 is shown, which is an injector for a liquid medication. The medication delivery device 5 comprises a housing 10, wherein a cartridge 6 containing a medical product is located. Through a needle unit (not shown) located at the distal end 12 of the medication delivery device 5, the medical product can be injected into a patient's skin using a piston (not shown) or the like, which can be moved into the distal direction with respect to the cartridge 6. The distal end 12 is covered by a cap 14 which may be removed before using the medication delivery device 5, thereby uncovering the needle.

For example, housing 10 can be integrally formed by several components like shells, which are attached to each other. In figure 1, the housing may have a tubular shape. It should be noted that medication delivery device 5 can be formed using various shapes, which can be selected according to operability needs or production costs, for example. Furthermore, housing 10 can include a window type cutout through which the cartridge 6 and, preferably, a position of the piston within the cartridge 6 are visible.

It should be noted that the description of medication injection device 5 as shown in figure 1 is merely illustrative. Other elements might be necessary in order to achieve full functionality. For example a dispense button and a drive mechanism can be present, which are configured to apply the adjusted dose value and move the piston in the distal direction such that the adjusted amount of the medical product is dispensed upon pressing the dispense button.

The medication delivery device 5 includes a window 8, which is arranged on the front face 18 near the proximal end 16 of the housing 10. The window 8 can be transparent, partially transparent or translucent, so as to allow a user of the medication delivery device 5 to look through the window 8 on underlying elements. The window 8 can be fabricated as a transparent sheet of plastic, for example.

The medication delivery device 5 includes a display element 20 being arranged on the front face 18 near the proximal end 16 of the housing 10. The display element 20 is located underneath the transparent window 8. Other embodiments, wherein the display element 20 directly forms the window 8, are also conceivable.
In the embodiment depicted in figure 1, the display element 20 is formed as a rectangular LCD-panel. The display element 20 is capable of displaying different types of information. For example, display element 20 indicates the dose to be administered which was set by the user.

Furthermore, status information relating to the medication delivery device can be provided. For example, the charging state of a battery used for operating device 5 or a residual amount of a medical product remaining in the cartridge for dispense can be displayed. Other information may include instruction information related to setting of a dose or delivering the dose.

As shown in figure 1, input means 22, 23, 24 are arranged on the front face 18 of the medication delivery device 5. Other locations of input means could be provided as well. For example, the input means can be configured to select the prescribed dose of medication which the patient wants to inject. In addition, the input means can be configured to enter further information, e.g. date and time of dose administration, about the type of medication contained in the cartridge or other data.

In the embodiment shown in figure 1, the input means are formed as buttons 22, 23, 24. It is conceivable, however, to use other type of input means, like sliders, turning and/or axially moving knobs or the like.

For dialling a specific dose value, a user selects the appropriate operating mode with button 22, for example. Furthermore, the dose value can be increased or decreased by pressing buttons 23 or 24, respectively. During this operation, the dose value can be displayed by the display element 20. It is also conceivable that the dose value is not affected by the user and is merely displayed for information. As such, buttons 22, 23, 24 can be used to enter other data like the time of applying the medicine or the like.

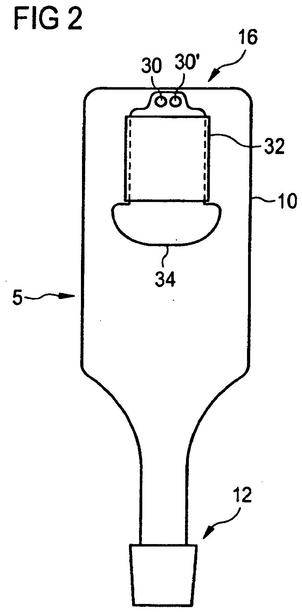

Making now reference to figure 2, a sectional drawing of a medication delivery device 5 is shown. Figure 2 depicts the medication delivery device 5 in a horizontal cut-off such that the components underneath the display element 20 and operating buttons 22, 23, 24 of figure 1 are visible.

As shown in figure 2, a light source 30 is arranged within the housing 10. According to an embodiment, the light source is a light-emitting-diode. The light source 30 is capable of providing an illumination of the display element 20 within the window 20 and at least one or all of the buttons 22, 23, 24.

The display element 20 can include an LCD-panel 32. The light source 30 provides backlight illumination to the LCD-panel 32. LCD-panel 32 can be fabricated from transparent substrates with polarization changing liquid crystals between the transparent substrates. Furthermore, the transparent substrate facing the front side 18 of the medication delivery device 5 can be covered by the transparent window 8. It is however also conceivable that the front side 18 facing transparent substrate itself forms the transparent window 8.

In the embodiment of figure 2, a light guide 34 is arranged underneath the LCD-panel 32. Furthermore, light guide 34 extends within the housing 10 to a position underneath the buttons 22, 23, 24. Light guide 34 performs light distribution from the light source 30 to the display element and the buttons 22, 23, 24.

As shown in figure 2, the light source 30 can be located near the proximal end 16 inside the housing 10 of the medication delivery device 5. Accordingly, light source 30 can be placed within the housing 10 at any suitable position essentially without constraints with respect to LCD-panel 32 and buttons 22, 23, 24. The light from the light source is distributed by the light guide 34 to LCD-panel 32 and buttons 22, 23, 24.

In the embodiment of figure 2, illumination of the LCD-panel 32 is provided by a transparent foil as light guide 34. The foil acts as a distributor for the light being emitted by the light source 30. The foil is arranged underneath LCD-panel 32 and extends to the operating buttons 22, 23, 24. In addition, the foil can be partly coated with a reflective layer, for example a metal layer. The reflective layer may enclose the transparent foil such that light can only escape at the positions of LCD-panel 32 and of buttons 22, 23, 24. Furthermore, a light diffusing element may be arranged on the foil at the position of LCD-panel 32 in order to provide a uniform illumination of the LCD-panel 32. In particular, no light source is required directly behind the display element and/or the buttons 22, 23, 24.

In order to allow the light to pass through the transparent window 8' of one or all of buttons 22, 23, 24 to be visible for a user, the respective button can be entirely formed using a transparent material. The respective button may be formed using a transparent or translucent material as the transparent window 8', for example. It is also conceivable, that an inlay of a transparent material is formed in the respective button or buttons as the transparent window 8'.

As shown in figure 2, a further light source 30' is arranged within the housing 10 next to light source 30. According to an embodiment, the further light source 30' is a light-emitting-diode. The further light source 30' is also capable of providing an illumination of the display element 20 and at least one of the buttons 22, 23, 24. Light source 30 and the further light source 30' may be adapted to provide illumination with different colors.

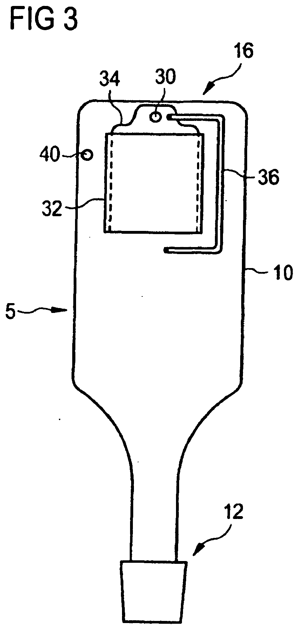

Making now reference to figure 3, a sectional drawing of another embodiment of a medication delivery device 5 is shown, which is currently not claimed. Figure 3 depicts medication delivery device 5 in a horizontal cut-off such that the components underneath the display 20 and operating buttons 22, 23, 24 of figure 1 are visible.

As shown in figure 3, light source 30 is arranged within the housing 10. The light source 30 is capable of providing an illumination of the display element 20 and at least one of the buttons 22, 23, 24. The light source 30 provides backlight illumination to the LCD-panel 32 by using the light guide 34, as described above in connection with figure 2.
In the embodiment of figure 3, an optical fiber 36 is arranged next to the light source 30 and extends to buttons 22, 23, 24 as a further light guide. Accordingly, light from the light source 30 may be coupled into the optical fiber and distributed by the optical fiber 36 to buttons 22, 23, 24.

As shown in figure 3, a further light source 40 is arranged within the housing 10 such that it is located next to the display element. Further light source 40 is used to provide further means for displaying information. According to an example, the further light source 40 is a light-emitting-diode. In order to allow the light emitted from the further light source 40 to be visible for a user from the outside, the housing 10 can include an optical transparent window, e.g. an inlay of a transparent material within a non-transparent section of the housing 10.

It should be noted that the display element 20 and input means can be illuminated independently. Therefore, it is conceivable that only the display element 20 or only at least one of buttons 22, 23, 24 is illuminated.

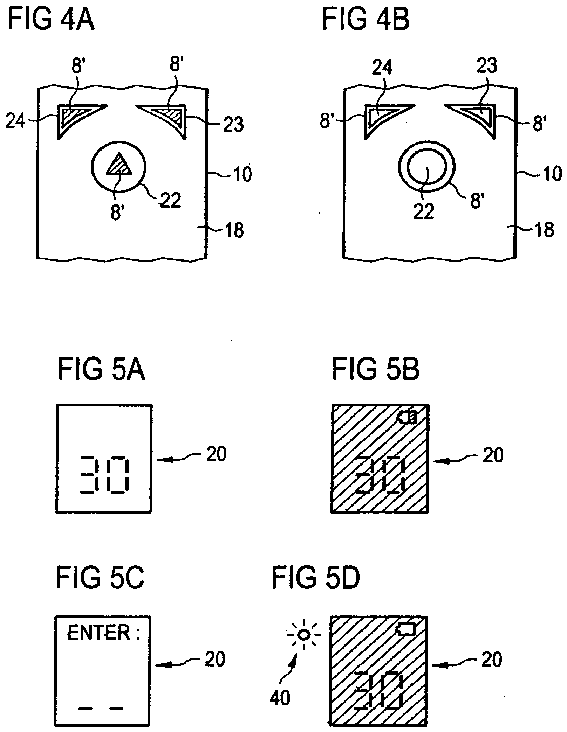

Illumination of the input means, i.e. the buttons for operating the medication delivery device 5, is now further described making reference to figures 4A and 4B which respectively show a front view of the medication delivery device 5 in an area around the input means.

In figure 4A, an embodiment of the medication delivery device 5 is shown. The medication delivery device 5 includes input means 22, 23, 24 which are arranged on the front face 18 of the medication delivery device 5. In the embodiment shown in figure 4A, the input means are formed as buttons 22, 23, 24. As mentioned above, other type of input means, like sliders, turning and/or axially moving knobs or the like can also be used.

According to the embodiment depicted in figure 4A, the buttons 22, 23, 24 are formed from a non light transparent material in which an inlay of a transparent material is formed as the transparent window 8'. The transparent window 8' allows the light to pass through one or all of buttons 22, 23, 24. In addition to be visible for a user, the inlay of the respective button can be formed in a symbol like fashion so that its function can be easily derived by the user. The respective button can also be formed using a transparent or translucent material for the button which acts as the transparent window 8'. In addition, a respective symbol can be printed on the front surface of one or all of buttons 22, 23 and 24.

In figure 4B, a further embodiment of the medication delivery device 5 is shown. The medication delivery device 5 includes input means 22, 23, 24 which are arranged on the front face 18 of the medication delivery device 5. In the embodiment shown in figure 4B, the input means are formed as buttons 22, 23, 24, for example.

According to the embodiment depicted in figure 4B, the buttons 22, 23, 24 are formed from a non light transparent material. The transparent window 8' is formed as a cut-out in housing 10, so as to provide a slit between the buttons 22, 23, 24 and the housing 10. The transparent window 8' allows the light from light source 30 and/or light source 30' (not shown in figure 4b) to pass through the transparent window 8' and thus illuminating the outline of one or all of buttons 22, 23, 24. Accordingly, buttons 22, 23, 24 are visible for a user, especially when operating medication delivery device 5 under dimly lit conditions or when operated by a visually impaired user. In addition, a transparent foil (not shown in figure 4B) can be provided on the front surface covering one or all of buttons 22, 23 and 24.

It should be noted that the embodiments as described with respect to figure 4A and 4B can also be mixed, i.e. be providing one button with a transparent inlay as the transparent window 8'and one with a surrounding slit as the transparent window 8'.

Making now reference to figures 5A to 5D, information referring to different types of operating modes is depicted on the display element 20.

As described above, the medication delivery device 5 can have different operating modes. Generally, the operating modes comprise at least a first state and a second state.

In figure 5A, as the first state a normal operating condition is depicted in which information is displayed on the display element. Here, white illumination of the display element 20 can be provided by the light source 30 as a first color, for example.

In figure 5B, as the second state an exceptional operating condition is depicted. Here light with a color other than white, preferably red or orange color, can be provided by the light source 30' in order to attract the attention of the user. The second state can be a low battery state of the medication delivery device 5, for example. It is also conceivable that during the exceptional operating condition, the light source 30' operates in a blinking mode.

In figure 5C, as another possible second state the input mode of the medication delivery device 5 is depicted, wherein a user input is required. Here light which provides illumination in a color other than white, e.g. green illumination, is emitted by the light source 30' in order to attract the attention of the user.

In figure 5D, as another possible second state another exceptional operating condition is depicted. Here, light with red or orange illumination is emitted by the light source 30' in order to attract the attention of the user. Furthermore, the further light source 40 next to the display element is activated as well.

Furthermore, it is also conceivable that a third state can be indicated for which a light source emits light with a different color with respect to the first and second state.

In general, an improved readability for the user is achieved as the light source illuminates the display element 20 and/or the operating buttons 22, 23, 24. Accordingly, operating the medication delivery device 5 is more convenient for the user. Furthermore, displayed information with higher brightness reduces the risk of misreading. By illuminating the operating buttons 22, 23, 24 and the display element 20, the operability of the medication delivery device 5 is enhanced, especially when operated by a visually impaired user.

Furthermore, the attention of the user is attracted with respect to the operating mode of the medication delivery device 5. As the illumination of the medication delivery device changes by applying different colors, a user can easily recognize the actual operating mode.

In addition, different colors assist the user during setup. This improves operability of the medication delivery device and allows for guiding the user through the various operating modes.

A method of manufacturing the medication delivery device 5 includes the steps of providing the housing 10 accommodating the cartridge 6 suitable for containing a medical product, the housing 10 having an at least partially transparent window 8 on a front surface 18 for displaying information. Furthermore, the light source 30 is arranged within the housing 10, wherein the light source 30 is adapted to provide an illumination of the at least part of the window 8.

### List of reference numerals:

Medication delivery device
   5
Cartridge
   6
Window
   8, 8'
Housing
   10
Distal end
   12
Cap
   14
Proximal end
   16
Front face
   18
Display element
   20
Buttons
   22, 23, 24
Light source
   30, 30'
LCD-panel
   32
Light guide
   34
Optical fiber
   36
Further light source
   40

## Claims

1. A medication delivery device comprising:
a housing (10) accommodating a cartridge suitable for containing a medical product,
the housing having a first at least partially transparent window (8) on a front surface (18) for displaying information, input means (22) for operating the medication delivery device wherein a second at least partially transparent window (8') is at least partially embedded in the input means on the front surface, and a light source (30, 30') being arranged within the housing (10), wherein the light source (30, 30') is adapted to provide an illumination of at least part of the first and second at least partially transparent windows (8, 8'),
wherein the device comprises a display element (20) within the first at least partially transparent window (8), and wherein the light source (30, 30') is capable of backlighting the display element (20),
**characterized in that** a light guide (34) is arranged in the optical path between the light source (30, 30') and the first and second at least partially transparent windows (8, 8'), wherein the light guide (34) comprises a foil wherein the foil is arranged underneath the display element (20) and the input means (22), wherein the light source (30, 30') is adapted to operate in a first state and in a second state. and wherein the second state corresponds to a configuration of the device requiring an input from a user or to an alarm state of the device.

2. The medication delivery device according to claim 1, wherein the light source (30, 30') comprises at least two light emitting elements capable of emitting light with different colors or brightness.

3. The medication delivery device according to claim 1 or 2, wherein the light source (30, 30') is adapted to provide light having a first color, preferably white or green, in the first state and the light source (30, 30') is adapted to provide light having a second color, preferably red or orange, in the second state.

4. The medication delivery device according to any of claims 3 4, wherein the light source (30, 30') is adapted to operate in a third state, in which the light source (30, 30') provides light with a different color with respect to the first and second color.

5. The medication delivery device according to any of claims 1 to 4, wherein the light source (30, 30') is adapted to provide a blinking illumination in the second state.

6. The medication delivery device according to any of claims 1 to 5, wherein the light source (30, 30') comprises light-emitting-diodes or a multi-color light-emitting-diode arrangement.

## Patentansprüche

1. Medikamentenabgabevorrichtung, die Folgendes aufweist:
ein Gehäuse (10) unterbringend eine Kartusche, die für das Aufnehmen eines medizinischen Produktes geeignet ist, wobei das Gehäuse ein erstes mindestens teilweise durchsichtiges Fenster (8) auf einer Vorderseite (18) zum Anzeigen von Informationen aufweist, Eingabemittel (22) zum Bedienen der Medikamentenabgabevorrichtung, wobei ein zweites mindestens teilweise durchsichtiges Fenster (8') mindestens teilweise in die Eingabemittel auf der Vorderseite eingebettet ist und eine Lichtquelle (30, 30'), die in dem Gehäuse (10) angeordnet ist, wobei die Lichtquelle (30, 30') angepasst ist, eine Beleuchtung von mindestens einem Teil des ersten und zweiten mindestens teilweise durchsichtigen Fensters (8, 8') zu liefern,
wobei die Vorrichtung ein Anzeigeelement (20) in dem ersten mindestens teilweise durchsichtigen Fenster (8) aufweist, und wobei die Lichtquelle (30, 30') zur Hintergrundbeleuchtung des Anzeigeelements (20) in der Lage ist,
**dadurch gekennzeichnet, dass** ein Lichtleiter (34) in dem Strahlengang zwischen der Lichtquelle (30, 30') und dem ersten und zweiten mindestens teilweise durchsichtigen Fenster (8, 8') angeordnet ist,
wobei der Lichtleiter (34) eine Folie umfasst, wobei die Folie unterhalb von dem Anzeigeelement (20) und den Eingabemitteln (22) angeordnet ist,
wobei die Lichtquelle (30, 30') angepasst ist, in einem ersten Zustand und
in einem zweiten Zustand betrieben zu werden, und wobei der zweite Zustand einer Konfiguration der Vorrichtung, die eine Eingabe von einem Anwender erfordert, oder einem Alarmzustand der Vorrichtung entspricht.

2. Medikamentenabgabevorrichtung gemäß Anspruch 1, wobei die Lichtquelle (30, 30') mindestens zwei Licht emittierende Elemente aufweist, welche in der Lage sind, Licht von unterschiedlicher Farbe oder unterschiedlicher Helligkeit auszustrahlen.

3. Medikamentenabgabevorrichtung gemäß Anspruch 1 oder 2, wobei die Lichtquelle (30, 30') angepasst ist, Licht mit einer ersten Farbe, vorzugsweise Weiß oder Grün, in dem ersten Zustand zu liefern und die Lichtquelle (30, 30') angepasst ist, Licht mit einer zweiten Farbe, vorzugsweise Rot oder Orange, in dem zweiten Zustand zu liefern.

4. Medikamentenabgabevorrichtung gemäß einem der Ansprüche 3, wobei die Lichtquelle (30, 30') angepasst ist, in einem dritten Zustand betrieben zu werden, in welchem die Lichtquelle (30, 30') Licht mit einer in Bezug zu der ersten und zweiten Farbe unterschiedlichen Farbe vorsieht.

5. Medikamentenabgabevorrichtung gemäß einem der Ansprüche 1 bis 4, wobei die Lichtquelle (30, 30') angepasst ist, eine blinkende Beleuchtung in dem zweiten Zustand zu liefern.

6. Medikamentenabgabevorrichtung gemäß einem der Ansprüche 1 bis 5, wobei die Lichtquelle (30, 30') lichtemittierende Dioden oder eine mehrfarbige Lichtemittierende-Dioden-Anordnung aufweist.

## Revendications

1. Dispositif de délivrance de médicament comprenant :
un boîtier (10) accueillant une cartouche appropriée pour contenir un produit médical, le boîtier ayant une première fenêtre au moins partiellement transparente (8) sur une surface avant (18) pour afficher des informations, un moyen d'entrée (22) pour faire fonctionner le dispositif de délivrance de médicament, où une deuxième fenêtre au moins partiellement transparente (8') est au moins partiellement intégrée dans le moyen d'entrée sur la surface avant, et une source de lumière (30, 30') étant disposée au sein du boîtier (10), où la source de lumière (30, 30') est adaptée pour fournir un éclairage d'au moins une partie des première et deuxième fenêtres au moins partiellement transparentes (8, 8'),
où le dispositif comprend un élément d'affichage (20) dans la première fenêtre au moins partiellement transparente (8), et où la source de lumière (30, 30') est capable de rétroéclairer l'élément d'affichage (20),
**caractérisé en ce qu'**un guide de lumière (34) est disposé au sein du chemin optique entre la source de lumière (30, 30') et les première et deuxième fenêtres au moins partiellement transparentes (8, 8'),
où le guide de lumière (34) comprend une feuille, où la feuille est disposée sous l'élément d'affichage (20) et le moyen d'entrée (22), où la source de lumière (30, 30') est adaptée pour fonctionner dans un premier état et dans un deuxième état, et où le deuxième état correspond à une configuration du dispositif requérant une entrée d'un utilisateur ou à un état d'alarme du dispositif.

2. Dispositif de délivrance de médicament selon la revendication 1, dans lequel la source de lumière (30, 30') comprend au moins deux éléments émetteurs de lumière capables d'émettre de la lumière de différentes couleurs ou luminosités.

3. Dispositif de délivrance de médicament selon la revendication 1 ou 2, dans lequel la source de lumière (30, 30') est adaptée pour fournir une lumière ayant une première couleur, de préférence du blanc ou du vert, dans le premier état et la source de lumière (30, 30') est adaptée pour fournir une lumière ayant une deuxième couleur, de préférence du rouge ou du orange, dans le deuxième état.

4. Dispositif de délivrance de médicament selon l'une quelconque des revendications 3, dans lequel la source de lumière (30, 30') est adaptée pour fonctionner dans un troisième état, où la source de lumière (30, 30') fournit une lumière d'une couleur différente des première et deuxième couleurs.

5. Dispositif de délivrance de médicament selon l'une quelconque des revendications 1 à 4, dans lequel la source de lumière (30, 30') est adaptée pour fournir un éclairage clignotant dans le deuxième état.

6. Dispositif de délivrance de médicament selon l'une quelconque des revendications 1 à 5, dans lequel la source de lumière (30, 30') comprend des diodes électroluminescentes ou un arrangement de diodes électroluminescentes multicolores.
